# EUROPEAN PATENT APPLICATION

(11) **EP 4 670 498 A1**
(43) Date of publication of application: **31.12.2025**
(21) Application number: 23924152.4
(22) Date of filing: 05.10.2023
(51) Int. Cl.: A01K 23/00, A61F 13/49, A61F 13/56

(54) **ABSORBENT ARTICLE FOR PET**

(30) Priority: 22.02.2023 JP 2023026287
(71) Applicant: DAIO PAPER CORPORATION, Shikokuchuo-shi Ehime 799-0492 (JP)
(72) Inventor: HONJO, Ryota, Shikokuchuo-shi, Ehime 799-0113 (JP)
(74) Representative: Epping - Hermann - Fischer
(86) International application number: PCT/JP2023/036359
(87) International publication number: WO 2024/176508

(57) **Abstract**

There are provided a dorsal region (R) covering a dorsal side from a base of a tail, a buttock covering region (M) covering an anus and a vicinity of a lower side thereof from the base of the tail, a ventral region (F) covering an abdomen on a front side of the buttock covering region (M), and an absorber (30) disposed at a middle portion in a width direction (D2), wherein the ventral region (F) has a belly wrap region (FA) joined to the dorsal region (R) at both ends in the width direction (D2), and is provided with extendable members (13) extending and contracting in the width direction (D2) over the whole area in a front-rear direction (D1) of the belly wrap region (FA). Hook tapes (12a, 12b) are provided at respective ends of the belly wrap region (FA) in the width direction (D2), a target tape (40) is provided at the dorsal region (R), and the hook tapes (12a, 12b) and the target tape (40) can be repeatedly joined to and separated from each other.

## Description

### FIELD OF THE INVENTION

The present disclosure relates to an absorbent article for pets.

As diapers for humans, two types of diapers are known, which are a tape type that is fixed with tape and a pants type that can be worn like pants. Of these, the pants-type diaper can be worn like pants, so it can be worn more easily than the tape-type diaper and has a fit like pants. Therefore, generally, the tape-type diaper is used for a while after birth, and the pants-type diaper is used when the pet becomes able to walk.

Patent Document 1 discloses a pants-type garment having a crotch portion, a waist portion, a left end engaging member, a right end engaging member, and an engaged region. The crotch portion has a crotch region, a front region on the front side of the crotch region, and a rear region on the rear side of the crotch region. The center region of the waist portion is joined to the rear viewing region. The left end engaging member is provided at the left end portion of the waist portion, and the right end engaging member is provided at the right end portion of the waist portion. The engaged region is provided at the front region and is engaged with the left end engaging member and the right end engaging member.

### Related Art Document

### Patent Document

Patent Document 1: Japanese Patent Laid-Open Publication No. 2022-14368

### SUMMARY OF THE INVENTION

### PROBLEM TO BE SOLVED BY THE INVENTION

In recent years, disposable diapers for pets such as dogs and cats have been known. However, as disposable diapers for pets, the above-mentioned tape-type diapers are known, but pants-type diapers are not known. The diaper disclosed in Patent Document 1 is a pants-type diaper, but it cannot be used for pets because it is for humans.

In addition, it is preferable to be able to appropriately select between a tape type and a pants type according to the breed and size of the pet.

It is an object of the present disclosure to provide an absorbent article for pets which can be used as both a pants type and a tape type.

### MEANS FOR SOLVING THE PROBLEM

In order to achieve the above object, an absorbent article for pets of a first embodiment of the present disclosure includes:
a dorsal region covering a dorsal side from a base of a tail;
a buttock covering region that is connected to the dorsal region in a first direction and covers an anus and a vicinity on a lower side thereof below the base of the tail;
a ventral region that is connected to the buttock covering region on a side opposite the dorsal region in the first direction, and covers an abdomen in front of the buttock covering region; and
an absorber disposed in a middle portion at least of the buttock covering region and the ventral region in a second direction orthogonal to the first direction,
wherein the ventral region has a belly wrap region that is joined to the dorsal region at both ends in the second direction, and is provided with extendable members that each extend and contract in the second direction, over an entire area of the belly wrap region in the first direction, and joining members that are joined to the dorsal region and provided at respective ends of the belly wrap region in a longitudinal direction,
the dorsal region is provided with a member to be joined to the joining members,
the joining members and the member to be joined are configured to be capable of being joined to and separated from each other repeatedly,
the joining members are configured to be capable of being repeatedly joined to and separated from even a portion of the belly wrap region to which the joining members are not attached.

According to the first embodiment, in a state where the joining member and the member to be joined are bonded, both ends in the second direction of the belly wrap region of the ventral region are bonded to the dorsal region, respectively, so that a waist opening for passing the trunk of the pet and a pair of left and right leg openings for passing the rear legs are formed. Further, an extendable member extending and contracting in the second direction is provided over the entire area of the belly wrap region in the first direction. Thus, it can be used as a pants type for obtaining a fit. Further, both ends in the second direction of the belly wrap region and the dorsal region are bonded so that they can be repeatedly bonded and separated from each other by the joining member provided in the belly wrap region and the member to be joined provided in the dorsal region. Therefore, it can also be used as a tape type by wearing the belly wrap region with the joining member and the member to be joined separated and then bonding them. Moreover, since the joining member is configured so that it can be repeatedly bonded and separated to the region of the belly wrap region where the joining member is not attached, the joining member can be bonded to the member to be joined for a large pet and the joining member can be bonded to the belly wrap region for a small pet.

In the second embodiment of the present disclosure, the belly wrap region has a length of 80 mm or more in the first direction.

According to the second embodiment, the width at which the belly wrap region and the dorsal region are joined is widened, thereby enhancing the fit of the pants type.

In the third embodiment of the present disclosure, the absorber is not disposed in the dorsal region.

According to the third embodiment, the cost can be reduced by not arranging the absorber in the dorsal region, since the absorber is not expected to have an effect in the dorsal region.

In the fourth embodiment of the present disclosure, the absorber is disposed such that an end portion of the absorber on a ventral region side is within a range of 10 mm to 40 mm from an end portion of the absorbent article for pets on a ventral region side.

According to the fourth embodiment of the present disclosure, the absorber can surely cover the urination opening even for a male pet whose urination opening is located on the ventral side, so that it can be adapted to a male pet.

A fifth embodiment of the present disclosure further comprises a pair of three-dimensional gathers that extend along respective sides of the absorber in the first direction and that rise toward a skin surface,
wherein a rising end portion of each of the three-dimensional gathers on a ventral region side is located within a range of 0 mm to 5 mm toward an end portion of the absorbent article for pets on a ventral region side and within a range of 0 mm to 5 mm toward an end portion of the absorbent article for pets on a dorsal region side, from an end portion of the absorber on a ventral region side.

According to the fifth embodiment, when a male pet wears the absorbent article for pets, a deformed protrusion protruding from the ventral region side end portion of the absorbent article toward the non-skin surface side is formed, thereby forming a urine capturing space further forward and effectively using the absorbent article to capture urine from the ventral region side end. Thus, leakage of urine to the outside can be suppressed.

In the sixth embodiment of the present disclosure, the rising end portion of the solid gather on a dorsal region side is located 100 mm to 200 mm closer to the end portion of the absorbent article for pets on the ventral region side than the end portion of the absorber on a dorsal region side is.

According to the sixth embodiment, when a female pet wears the absorbent article for the pet, the three-dimensional gathers stand up against the skin side from the ventral side of the female pet to both sides of the urination opening. Thus, even when the female pet wears the absorbent article for the pet, leakage of urine to the outside can be suppressed.

According to the seventh embodiment of the present disclosure, the belly wrap region includes a first region overlapping the absorber and a second region not overlapping the absorber, and
the second region is extended and contracted in the second direction by the extendable members, and the first region is not extended and contracted in the second direction.

According to the seventh embodiment, when the male pet wears the absorbent article for the pet, the second region of the belly wrap region extends and contracts in the second direction, so both ends of the absorbent article in the second direction contract inwardly and adhere to the abdomen of the male pet. On the other hand, since the first region overlapping the absorbent article does not extend and contract in the second direction on the absorbent article, the absorbent located in the first region does not adhere to the abdomen of the male pet and is loose. Thus, a urine capturing space is formed in which the absorbent surrounds the front and the periphery of the urination opening of the male pet at a distance. Therefore, urine can be sufficiently captured by the absorber, and leakage of urine to the outside can be suppressed. Further, since the urination opening and the absorber are separated from each other, the urine absorbed by the absorber can be suppressed from coming into contact with and adhering to the urination opening.

In the eighth embodiment of the present disclosure, the extendable members are capable of extending to be 1.5 to 3.5 times the length of the extendable members when contracted, in the second direction of the belly wrap region.

According to the eighth embodiment of the present disclosure, a well-fitting feeling of the pants type can be further obtained.

In the ninth embodiment of the present disclosure, the ventral region includes only the belly wrap region provided with the extendable members and the joining members.

According to the ninth embodiment of the present disclosure, while forming the pants type, the bulkiness of wearing the absorbent article for pets is suppressed by shortening the length of the ventral region in the first direction, thereby improving the slim feeling and improving the appearance.

In the tenth embodiment of the present disclosure, the joining members are provided over the entire area of the dorsal region in the second direction.

According to the tenth embodiment, the joining members can be joined at any position in the second direction of the dorsal region, and the waist opening can be easily adjusted according to the waist of the pet.

In the eleventh embodiment of the present disclosure, the joining members are joined to the member to be joined, to form a pants type.

According to the eleventh embodiment, the pants type can be provided.

According to the twelfth embodiment of the present disclosure, the joining members are joined to a region, of the member to be joined, corresponding to the body shape of a pet wearing the absorbent article for pets, to form a pants type.

According to the twelfth embodiment, the pants type can be selected according to the body shape of the pet wearing the absorbent article for pet.

### Effect of the Invention

According to the present disclosure, the absorbent article for pets can be used as both the pants type and the tape type.

### BRIEF DESCRIPTION OF THE DRAWINGS

[FIG. 1] FIG. 1 is a view of an embodiment of the absorbent article for pets of the present disclosure viewed from the skin side.
[FIG. 2] FIG. 2 is a cross-sectional view along A-A shown in FIG. 1.
[FIG. 3] FIG. 3 is a cross-sectional view along B-B shown in FIG. 1.
[FIG. 4] FIG. 4 is a cross-sectional view along C-C shown in FIG. 1.
[FIG. 5] FIG. 5 is a diagram illustrating a mode of use of the diaper shown in FIG. 1.
[FIG. 6] FIG. 6 is a diagram illustrating one mode of using the diaper shown in FIG. 1 as a pants type.
[FIG. 7] FIG. 7 is a diagram illustrating one mode of using the diaper shown in FIG. 1 as a tape type.
[FIG. 8] FIG. 8 is a diagram illustrating an effect when the diaper shown in FIG. 1 is used on a male pet.
[FIG. 9] FIG. 9 is a diagram illustrating another mode of use of the diaper shown in FIG. 1, with (a) being an external perspective view and (b) showing (a) as viewed along a direction of an arrow E.
[FIG. 10] FIG. 10 is a view of another embodiment of the absorbent article for pets of the present disclosure viewed from the skin side.
[FIG. 11] FIG. 11 is a view of another embodiment of the absorbent article for pets of the present disclosure viewed from the skin side.

### DETAILED DESCRIPTION OF THE INVENTION

Embodiments of the present disclosure will be described below with reference to the drawings. In each of the drawings, unless otherwise explained, the same or corresponding configurations may be denoted by the same reference numerals and their explanation omitted. In addition, for the directions of orthogonal, lateral, and the like, deviations to the extent that they do not impair the action and effect of the embodiment are allowed. The shape of the corners is not limited to a right angle, and may be rounded in a bow shape. Orthogonal may include substantially orthogonal. In this specification, the longitudinal direction of a diaper 1 is referred to as D1, and the width direction of the diaper 1 orthogonal to the longitudinal direction is referred to as D2. The longitudinal direction corresponds to the longitudinal direction of the pet. In this specification, the front side of the diaper 1 in the longitudinal direction is referred to as ventral or anterior, and the rear side of the diaper 1 in the longitudinal direction is referred to as dorsal or posterior. The surface of the diaper 1 facing the pet is referred to as skin surface, and the surface opposite to the skin surface of the diaper 1 is referred to as non-skin surface.

### (Overall Structure)

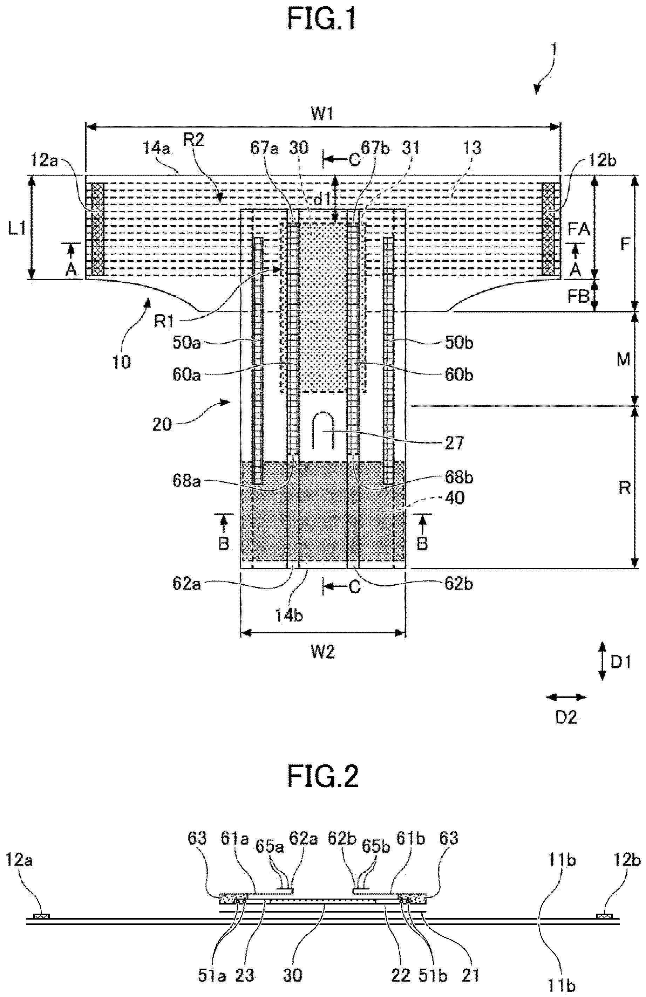

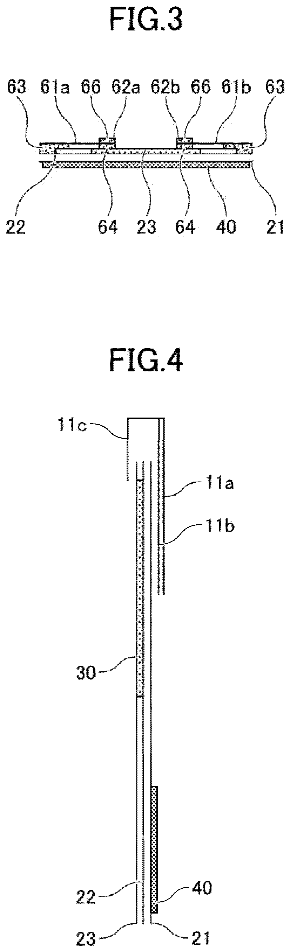

FIG. 1 is a view of an embodiment of the absorbent article for pets of the present disclosure viewed from the skin side, and is a plan view in a laid-out state. FIG. 2 is a cross-sectional view along A-A shown in FIG. 1. FIG. 3 is a cross-sectional view along B-B shown in FIG. 1. FIG. 4 is a cross-sectional view along C-C shown in FIG. 1.

In the present embodiment, as shown in FIG. 1, the diaper 1 is configured such that the dorsal region R, the buttock covering region M, and the ventral region F are connected to each other in this order in the front-rear direction D1, which is the first direction. The dorsal region R covers the dorsal side from the base of the tail of the pet wearing the diaper 1. The buttock covering region M covers the anus and the vicinity of the lower side thereof, below the base of the tail of the pet wearing the diaper 1. The ventral region F covers the abdomen on the front side of the pet wearing the diaper 1, more than the buttock covering region M. In the ventral region F, the belly wrap region FA and the leg circumference region FB are aligned in the front-rear direction D1, and the belly wrap region FA is on the side of the ventral end portion 14a of the diaper 1 in the front-rear direction D1 of the diaper 1.

The diaper 1 has an exterior body 10 and an interior body 20. The exterior body 10 is arranged only in the ventral region F, and the interior body 20 is arranged in a part of the ventral region F, as well as in the dorsal region R and the buttock covering region M. The length W1 of the exterior body 10, in the width direction D2 which is the second direction, is longer than the length W2 of the interior body 20 in the width direction D2. Thus, the length of the ventral region F of the exterior body 10 in the width direction D2 is longer than the length of the dorsal region R and the buttock covering region M of the exterior body 10 in the width direction D2. Specifically, it is conceivable that the exterior body 10 has a length of 165 mm in the front-rear direction D1 and a length of 600 mm in the width direction D2, which is the second direction. It is conceivable that the interior body 20 has a length of 410 mm in the front-rear direction D1 and a length of 190 mm in the width direction D2. The interior body 20 is positioned overlaying a part of the exterior body 10 at the center in the width direction D2 of the interior body 20. For example, it is conceivable that a region of 135 mm of the interior body 20 on the ventral side in the front-rear direction D1 is positioned overlaying the exterior body 10 so as to be placed on the skin surface of the exterior body 10. Thus, in the diaper 1, the exterior body 10 and the interior body 20 are positioned overlapping each other in a part of the ventral region F to form a T-shape as a whole. In this case, the length thereof in the front-rear direction D1 is 440 mm, and the length thereof in the width direction D2 is 600 mm. The term "length" here means the length of the diaper 1 when the diaper 1 is laid-out and stretched to a maximum. The length of the diaper 1 in the front-rear direction D1 is preferably 300 mm to 600 mm, the length of the exterior body 10 in the width direction D2 is preferably 500 mm to 800 mm, and the length of the interior body 20 in the width direction D2 is preferably 100 mm to 250 mm. Thus, when the pet wears the diaper 1, the diaper 1 can be fitted around the legs and waist.

### (Exterior Body 10)

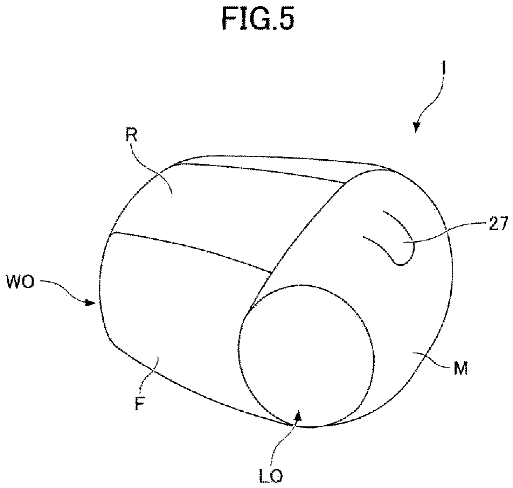

The exterior body 10 forms the ventral region F along with a portion of the interior body 20 positioned overlaying the exterior body 10. In the belly wrap region FA of the ventral region F, the exterior body 10 has a belt-like shape having the width direction D2 as the longitudinal direction. In the leg circumference region FB of the ventral region F, both ends of the exterior body 10 in the width direction D2 are cut in a curved shape so that a part of the leg opening LO (see FIG. 5) is formed when a pet wears the diaper 1. As a result, the exterior body 10 has a shape in which the length of the leg circumference region FB in the width direction D2 gradually becomes shorter as the leg circumference region FB becomes farther from the belly wrap region FA.

The exterior body 10 is composed of two exterior sheets 11a and 11b bonded together by an adhesive such as a hot-melt adhesive. The adhesive may be used to partially bond together each of the two exterior sheets 11a and 11b may be bonded together by the adhesive. Of the two exterior sheets 11a and 11b, the exterior sheet 11a disposed on the outside is folded inward at the ventral end portion of the diaper 1 in the front-rear direction D1, and has a folded portion 11c. The folded portion 11c partially covers the interior body 20.

Anything in the form of sheets can be used as the two exterior sheets 11a and 11b without any particular limitation, but it is preferable to use a nonwoven fabric. The raw fiber of the nonwoven fabric is not particularly limited. For example, synthetic fibers such as olefinic fibers, such as polyethylene or polypropylene, polyester fibers, or polyamide fibers; recycled fibers such as rayon or cupra; natural fibers such as cotton; or mixed fibers or composite fibers using two or more of these fibers can be used. Furthermore, the nonwoven fabric may be manufactured by any processing.

A plurality of extendable members 13 are sandwiched between the two exterior sheets 11a and 11b in the belly wrap region FA. It is conceivable that something long and thin can be used as an extendable member 13. The extendable members 13 extend in a direction of extension and contraction in the width direction D2, between both ends of the exterior body 10 in the width direction D2, and are fixed between the two exterior sheets 11a and 11b in a state of being parallel to each other, with a space between each other in the front-rear direction D1.

The extendable members 13 are arranged from a region, which is 0 mm to 5 mm from the ventral end portion 14a of the diaper 1, toward the dorsal side of the diaper 1. It is conceivable that the extendable members 13 are arranged up to a region that is 0 mm to 5 mm from the dorsal end portion of the belly wrap region FA in the front-rear direction D1. As described above, the extendable members 13 are not limited to the case where they are provided between both ends of the belly wrap region FA in the front-rear direction D1, and may be provided without being included in regions within 5 mm from each end portion of the belly wrap region FA in the front-rear direction D1. In either case, it is defined as the extendable members 13 being provided over the entire area of the belly wrap region FA in the front-rear direction D1. Instead, in this case, the plurality of extendable members 13 may be partially provided at locations over the whole area of the belly wrap region FA in the front-rear direction D1. The extendable members 13 may be fixed to the exterior sheets 11a and 11b by hot-melt bonding, heat sealing, ultrasonic bonding, or the like. It is not necessary that all extendable members 13 have the same thickness and extension ratio, but for example, the thicknesses and extension ratios may be different between those on the end portion 14a side of the diaper 1 and those on the leg circumference region FB side. Here, a part of the interior body 20 is positioned overlaying the exterior body 10 as described above. At this time, the exterior body 10 has, in the belly wrap region FA, a first region R1 which overlaps the absorber 30 of the interior body 20 and a second region R2 which does not overlap the absorber 30. In the belly wrap region FA, each extendable member 13 is sandwiched between the two exterior sheets 11a and 11b, so that it extends and contracts in the width direction D2, and even though the second region R2 extends and contracts in the width direction D2, the first region R1 does not extend and contract in the width direction D2. This configuration may be realized by cutting the extendable members 13 only in the first region R1 using a shirring cutter. Moreover, even if only because of the overlap with the absorber 30, the exterior body 10 has difficulty extending and contracting in the width direction D2 in the first region R1.

Examples of the extendable member 13 include yarn rubber, an elastic film, a member formed by sticking a nonwoven fabric to these materials, and the like. Styrene-based rubber, olefin-based rubber, urethane-based rubber, ester-based rubber, polyurethane, polyethylene, polystyrene, styrene-butadiene, silicon, polyester, and the like that are usually used can be used as the material constituting the extendable members 13.

The exterior body 10 has hook tapes 12a and 12b attached to respective ends in the width direction D2, in the belly wrap region FA. The hook tapes 12a and 12b are an example of a joining member in the present disclosure, have a belt-like shape, and are attached to the exterior body 10 while being in a direction by which the longitudinal direction thereof coincides with the front-rear direction D1 of the diaper 1. That is, the length of the hook tapes 12a and 12b in the longitudinal direction thereof can be appropriately set as long as it is less than the length of the exterior body 10 in the front-rear direction D1, in the belly wrap region FA. The surfaces of the hook tapes 12a and 12b are composed of a male material of a surface fastener, and the hook tapes 12a and 12b are fixed to the surface of the exterior body 10 on which the interior body 20 is superimposed, by an adhesive or the like.

### (Interior body 20)

The interior body 20 forms a part of the ventral region F, as well as the buttock covering region M and the dorsal region R. The interior body 20 has a back sheet 21, a waterproof sheet 22, a top sheet 23, the absorber 30, a pair of planar gathers 50a and 50b, three-dimensional gathers 60a and 60b, and a target tape 40.

The back sheet 21 has the same outer shape as the interior body 20, and is arranged on the most non-skin-side of the interior body 20. A nonwoven fabric is used as the back sheet 21. As the constituent fibers of the nonwoven fabric, for example, synthetic fibers (in addition to single component fibers, composite fibers such as core sheaths are also included) such as olefinic fibers including polyethylene or polypropylene; polyester fibers and polyamide fibers; recycled fibers such as rayon or cupra; natural fibers such as cotton; and the like, can be selected without particular limitation, and a mixture of these fibers can be used. Specifically, as the nonwoven fabric, spunbond nonwoven fabric, meltblown nonwoven fabric, spunlace nonwoven fabric, thermal bond (air-through) nonwoven fabric, needle punch nonwoven fabric, point bond nonwoven fabric, laminated nonwoven fabric (SSS (spunbond + spunbond + spunbond) nonwoven fabric in which the same or similar nonwoven fabric layers are laminated), and the like may be used. The fiber weight of the back sheet 21 is preferably 10 to 50 g/m², more preferably 15 to 30 g/m².

The target tape 40 is attached to the non-skin surface of the back sheet 21. The target tape 40 is an example of a member to be joined in the present disclosure. The target tape 40 has a rectangular shape and is attached along the end portion 14b of the diaper 1 in the dorsal region R. The length of the target tape 40 in the width direction D2 may be 180 mm, for example, when the length of the back sheet 21 in the width direction D2 is 190 mm. As described above, it is preferable that the target tape 40 is attached over almost the entire area of the back sheet 21 in the width direction D2. Including this case, the target tape 40 is defined as being attached over the entire area of the back sheet 21 in the width direction D2. The surface of the target tape 40 is composed of a female material with a surface fastener, and is fixed to the non-skin surface of the back sheet 21 by an adhesive or the like.

The waterproof sheet 22 is an impervious sheet that does not allow excretory fluid to permeate, and is laminated on the skin surface side surface of the back sheet 21 and adhered thereto by an adhesive or the like. As the waterproof sheet 22, for example, a microporous sheet obtained by kneading an inorganic filler in an olefinic resin such as polyethylene or polypropylene, molding the sheet, and then stretching it in a uniaxial or biaxial direction can be suitably used. In addition, as the waterproof sheet 22, a nonwoven fabric with an enhanced waterproof property may be used as a base material. The waterproof sheet 22 has the same outer shape as the interior body 20, but preferably at least extends more widely than the absorber 30 in the front-rear direction D1 and the width direction D2.

The absorber 30 is provided on the waterproof sheet 22 so as to be included in the ventral region F and the buttock covering region M at a middle portion in the width direction D2 of the diaper 1. The absorber 30 may be slightly displaced in the width direction D2 from the middle portion of the diaper 1 in the width direction D2, and even in this case, it is defined as being provided at the middle portion of the diaper 1 in the width direction D2. In addition, it is preferable that the absorber 30 is disposed so that the distance d1 from the end portion 31 on the ventral region F side of the absorber 30 to the ventral end portion 14a of the diaper 1 is in the range of 5 mm to 50 mm. Further, it is preferable that the absorber 30 is disposed so that the distance d1 of the absorber 30 is in the range of 10 mm to 40 mm. Thus, when the diaper 1 is worn by a male pet, the absorber 30 covers the urination opening of the male pet, and can be adapted to the male pet. The absorber 30 may be wrapped with a wrapping sheet made of crepe paper or nonwoven fabric for the purpose of maintaining its shape. The absorber 30 is a part that absorbs and holds excretory fluid, and can be formed of an aggregate of fibers. As the fiber aggregate, a fiber aggregate obtained by stacking short fibers such as cotton-like pulp, synthetic fibers, and natural fibers made of softwood or hardwood, and a filament aggregate obtained by opening synthetic fiber tow (fiber bundle), such as cellulose acetate and the like as necessary can be used. The fiber weight can be, for example, about 100 to 300 g/m² when stacking cotton-like pulp or short fibers, and it can be, for example, about 30 to 120 g/m² when stacking filament aggregate. In the case of synthetic fibers, the fineness is preferably 1 to 16 dtex, more preferably 1 to 10 dtex, and more preferably 1 to 5 dtex. A part or all of the absorber 30 may contain highly absorbent polymer particles. The highly absorbent polymer particles include powder as well as particles. As the highly absorbent polymer particles, materials used for such absorbent articles can be used as they are. As the material of the highly absorbent polymer particles, for example, starch-based materials, cellulose-based materials, synthetic polymer-based materials, and the like can be used. In addition, the absorber 30 may have a shape, in a plan view, in which the central part in the front-rear direction D1 is constricted. In other words, the absorber 30 may have a configuration in which the length in the width direction D2 gradually decreases toward the center of the front-rear direction D1.

The top sheet 23 is a liquid-permeable sheet that is quickly penetrated by excretory fluid such as urine. The top sheet 23 has the same length as the interior body 20 in the front-rear direction D1 of the diaper 1, and extends farther laterally than the absorber 30 in the width direction D2 to cover the absorber 30 and the planar gathers 50a and 50b. The top sheet 23 is laminated on the waterproof sheet 22 so as to cover the absorber 30 and the planar gathers 50a and 50b, and is bonded to the waterproof sheet 22. Thus, the absorber 30 is sandwiched between the waterproof sheet 22 and the top sheet 23. For example, when the rising end portions of the three-dimensional gathers 60a and 60b, which will be described later, are located in the center of the side edge of the absorber 30 in the width direction D2, the length of the top sheet 23 in the width direction D2 may be made shorter than the length of the absorber 30 in the width direction D2.

A porous or non-porous nonwoven fabric or a porous plastic sheet is suitably used as the top sheet 23. As the constituent fibers of the nonwoven fabric, for example, synthetic fibers (in addition to single component fibers, composite fibers such as core sheaths are also included) such as olefinic fibers including polyethylene or polypropylene; polyester fibers such as polyamide; recycled fibers such as rayon or cupra; natural fibers such as cotton; and the like can be selected without particular limitation, and any of these fibers can be mixed. In order to enhance the flexibility of the nonwoven fabric, it is preferable that the constituent fibers be crimped fibers. The constituent fibers of the nonwoven fabric may be hydrophilic fibers (including hydrophilic fibers made hydrophilic by a hydrophilizing agent), hydrophobic fibers or water-repellent fibers (including water-repellent fibers made water-repellent by a water-repellent agent).

The nonwoven fabric is generally classified into short nonwoven fabric, long nonwoven fabric, spunbond nonwoven fabric, meltblown nonwoven fabric, spunlace nonwoven fabric, thermal bond (air-through) nonwoven fabric, needle punch nonwoven fabric, point bond nonwoven fabric, laminated nonwoven fabric (SSS (spunbond + spunbond + spunbond) nonwoven fabric in which the same or similar nonwoven fabric layers are laminated), and additionally as SMS (spun-bond + melt blown + spun-bond) nonwoven fabric, SMMS (spun-bond + melt blown + melt blown + spun-bond) nonwoven fabric, and the like in which different nonwoven layers are laminated and melt blown layers are sandwiched between spun-bond layers), and the like, depending on the fabric length, sheet forming method, fabric bonding method, and lamination structure. Preferably, the laminated nonwoven fabric is manufactured as an integral nonwoven fabric including all the layers and subjected to fiber bonding over all the layers. As a result, it is possible to improve the feeling on skin, and therefore, it is possible to prevent the skin of the pet from being irritated when it comes into contact with the skin. The laminated nonwoven fabric may be formed by bonding a plurality of separately manufactured nonwoven fabrics by means of a hot-melt adhesive or the like.

The three-dimensional gathers 60a and 60b have a function of preventing the excrement traveling on the top sheet 23 and moving in the lateral direction and preventing the lateral leakage. The three-dimensional gathers 60a and 60b extend along both sides of the absorber 30 in the front-rear direction D1, and are formed by folding back two gather sheets 61a and 61b laminated on the top sheet 23. The two gather sheets 61a and 61b are arranged in the center of the diaper 1 in the width direction D2 at a predetermined interval, and the end sides facing each other are folded-back parts 62a and 62b. The length of the two gather sheets 61a and 61b in the front-rear direction D1 is the same as the length of the interior body 20, and the folded-back parts 62a and 62b are arranged so as to overlap the absorber 30. The two gather sheets 61a and 61b are arranged so that the ends opposite to the folded-back parts 62a and 62b, that is, the outer ends in the width direction D2, overlap the ends of the waterproof sheet 22 and the back sheet 21. The outer sides of the two gather sheets 61a and 61b in the width direction D2 are fixed to the top sheet 23 and the waterproof sheet 22 by an adhesive 63, over the entire length in the front-rear direction D1.

In addition, at the ventral end portion of the three-dimensional gathers 60a and 60b in the front-rear direction D1, the folded-back parts 62a and 62b are fixed to the top sheet 23 by an adhesive 64 and cannot be spread due to an adhesive 66. The boundary between the portion where the folded-back parts 62a and 62b are fixed to the top sheet 23 and the portion where they are not fixed becomes the rising end portions 67a and 67b on the ventral region F side of the three-dimensional gathers 60a and 60b. The rising end portions 67a and 67b of the three-dimensional gathers 60a and 60b are preferably located within a range of 0 mm to 5 mm toward the ventral end portion 14a side of the diaper 1 or within a range of 0 mm to 5 mm toward the dorsal end portion 14b side of the diaper 1, from the end portion 31 on the ventral region F side of the absorber 30.

In addition, at the dorsal end portion of the three-dimensional gathers 60a and 60b in the front-rear direction D1 and the region facing the target tape 40, the folded-back parts 62a and 62b are fixed to the top sheet 23 by the adhesive 64 and cannot be spread due to the adhesive 66. The boundary between the portion where the folded-back parts 62a and 62b are fixed to the top sheet 23 and the portion where they are not fixed becomes rising end portions 68a and 68b on the dorsal region R side of the three-dimensional gathers 60a and 60b. The rising end portions 68a and 68b are preferably located within a range of 100 to 200 mm toward the ventral end portion 14a side from the dorsal end portion 14b of the diaper 1.

The folded-back parts 62a and 62b include extendable members 65a and 65b extending in the front-rear direction D1, in a region not fixed to the top sheet 23 by the adhesive 64.

In the three-dimensional gathers 60a and 60b constructed in this way, an area which is not fixed to the top sheet 23 by the adhesive 64 can rise up toward the skin surface as the rising end portions 67a, 67b, 68a, and 68b at the portions fixed by the adhesive 63. Thus, the three-dimensional gathers 60a and 60b can rise up by utilizing the contraction force of the extendable members 65a and 65b. When the three-dimensional gathers 60a and 60b rise up, the three-dimensional gathers 60a and 60b can be brought into close contact with the body surface of the pet. In the example shown in FIG. 2, the folded-back parts 62a and 62b each contain two extendable members 65a and 65b, but the number of these members may be one. On the other hand, as shown in FIG. 3, both ends of the three-dimensional gathers 60a and 60b in the front-rear direction D1 are fixed to the top sheet 23 by the adhesive 64 as described above, so they do not rise up to the skin surface.

Hot-melt adhesives are used as the adhesives 63, 64, and 66. In this embodiment, adhesives 63, 64, and 66 are used as the fixing means, but any fixing means such as fusion, crimping, or the like may be used.

As the extendable members 65a and 65b, materials such as styrene-based rubber, olefin-based rubber, urethane-based rubber, ester-based rubber, polyurethane, polyethylene, polystyrene, styrene-butadiene, silicon, polyester, and the like which are normally used may be used.

As the gather sheets 61a and 61b, a nonwoven fabric obtained by a suitable processing method such as a spun-bond method, an air-through method, a melt-blown method, or a needle punch method can be used. The material fibers constituting the gather sheets 61a and 61b can be synthetic fibers such as olefinic fibers including polyethylene or polypropylene, polyester fibers, and amide fibers; recycled fibers such as rayon or cupra; and natural fibers such as cotton.

The planar gathers 50a an 50b are composed of extendable members 51a and 51b arranged between the top sheet 23 and the waterproof sheet 22 and fixed thereto. The extendable members 51a and 51b are arranged outside the three-dimensional gathers 60a and 60b and in parallel with the three-dimensional gathers 60a and 60b, in a region that does not overlap the absorber 30, and extend and contract in the front-rear direction D1. The ventral end portions of the planar gathers 50a and 50b are positioned in the ventral region F at locations closer to the dorsal side than the ventral rising end portions 67a and 67b of the three-dimensional gathers 60a and 60b, and the dorsal end portions of the planar gathers 50a and 50b are positioned in the dorsal region R in a region that overlaps the target tape 40. The planar gathers 50a and 50b hold the diaper 1 along the leg circumference to improve the fit and prevent the diaper 1 from shifting when the diaper is worn. A material similar to that of the extendable members 65a and 65b of the three-dimensional gathers 60a and 60b can be used as the extendable members 51a and 51b.

By arranging the planar gathers 50a and 50b in parallel with the three-dimensional gathers 60a and 60b, the planar gathers 50a and 50b can increase the elastic stretching force of the three-dimensional gathers 60a and 60b in the front-rear direction D1 of the diaper 1, and easily form a urine capturing space. In addition, since the diaper 1 can be held along the leg circumference to improve the fit, leakage of urine to the dorsal region B can be suppressed.

In the dorsal region B of the diaper 1, a tail opening 27 through which the tail passes is provided in a region where the target tape 40 is not provided. The tail opening 27 may be a slit penetrating in the thickness direction of the diaper 1, or a sewing machine eye point intermittently penetrating in the thickness direction of the diaper 1. The tail opening 27 may be provided in a U-shape. In this case, since the absorber 30 is not provided in the tail opening 27, the tail opening 27 can be easily formed.

### (Diaper Usage Mode)

FIG. 5 shows a usage mode of the diaper 1 shown in FIG. 1.

The diaper 1 shown in FIG. 1 is used as a pants type. In this case, as shown in FIG. 5, the diaper 1 is folded back so that the skin surface is inside in the buttock covering region M, and the skin surface at both ends of the ventral region F in the width direction D2 is opposed to the non-skin surface of the dorsal region R. Hook tapes 12a and 12b are attached to the skin surface at respective ends of the ventral region F in the width direction D2, and a target tape 40 is attached to the non-skin surface of the dorsal region R. Therefore, the hook tapes 12a and 12b are joined to the target tape 40, thereby joining the ventral region F and the dorsal region R.

Thus, the waist opening WO for passing the torso of the pet is formed by the belly wrap region FA of the ventral region F and the dorsal region R. Furthermore, the leg circumference region FB of the ventral region F and the buttock covering region M form a pair of left and right leg openings LO for passing the hind legs of the pet. At this time, the belly wrap region FA of the ventral region F tightens around the waist of the pet, and the belly wrap region FA is provided with the extendable members 13 extending in the width direction D2, over the whole area in the front-rear direction D1. Thus, the pants-type diaper 1 can obtain a fitting feeling like pants. In addition, if the hook tapes 12a and 12b are joined to a region of the target tape 40 corresponding to the body shape of the pet wearing the diaper 1, the pants type corresponding to the body shape of the pet wearing the diaper 1 can be selected. In addition, if the length of the belly wrap region FA in the front-rear direction D1 is 80 mm or more, the width where the belly wrap region FA and the dorsal region R are joined is widened, and the fit of the pants type can be further obtained.

In addition, if the extendable members 13 can be extended to be 1.5 to 3.5 times as long as the length thereof in the width direction D2 of the belly wrap region FA when the extendable members 13 are contracted, the fit of the pants type can be further obtained.

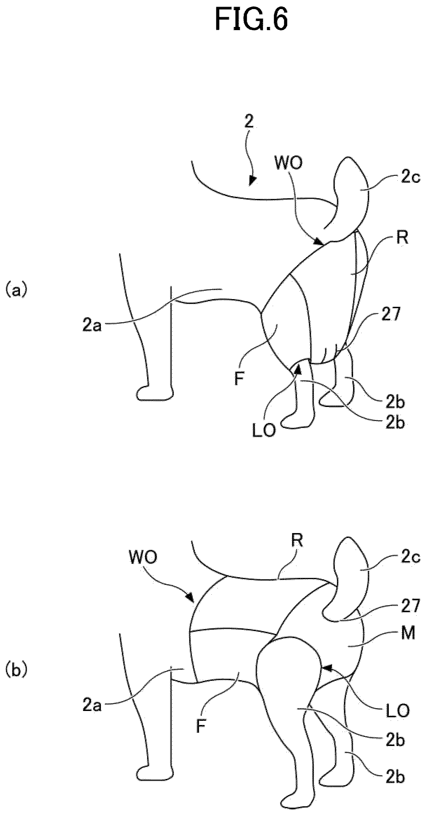

FIG. 6 is a diagram for explaining one embodiment of using the diaper 1 shown in FIG. 1 as the pants type.

The diaper 1 shown in FIG. 1 can be used as the pants type shown in FIG. 5.

In this case, as shown in FIG. 6(a), first, the legs 2b of a male dog 2, which is a pet, are passed through the waist opening WO formed by the ventral region F and the dorsal region R. Furthermore, the legs 2b of the male dog 2 are passed through the pair of leg openings LO formed by the leg circumferential region FB of the ventral region F and the buttock covering region M.

Then, the diaper 1 is moved to the ventral side of the male dog 2 while passing the trunk 2a of the male dog 2 through the waist opening WO so as to make the male dog 2 wear pants. As shown in FIG. 6(b), when the legs 2b of the male dog 2 have passed through the leg openings LO, the ventral region F is wrapped around the trunk 2a of the male dog 2, the buttock covering region M covers the buttocks of the male dog 2, and the dorsal region R covers the back of the male dog 2. The tail 2c of the male dog 2 is passed through the tail opening 27.

In this state, the extendable members 13 extending in the width direction D2 are provided over the whole area of the belly wrap region FA of the ventral region F in the front-rear direction D1, so that the pants-type diaper 1 can be fitted like pants.

In this way, the diaper 1 shown in FIG. 1 can be worn by a pet as the pants-type diaper shown in FIG. 5.

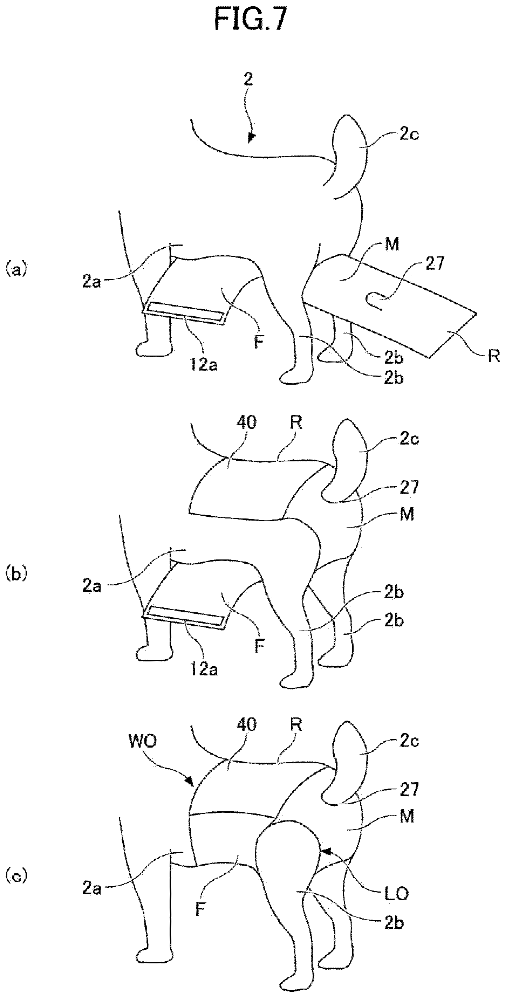

FIG. 7 is a view for explaining one embodiment in which the diaper 1 shown in FIG. 1 is used as a tape type.

The diaper 1 shown in FIG. 1 can be used as a tape type.

In this case, the hook tapes 12a and 12b and the target tape 40 are separated from the form shown in FIG. 5. As described above, the hook tapes 12a and 12b have surfaces of male material with surface fasteners, and the target tape 40 has surfaces of female material with surface fasteners. Therefore, the hook tapes 12a and 12b and the target tape 40 can be repeatedly joined and separated from each other. Thus, the hook tapes 12a and 12b and the target tape 40 can be separated from the form shown in FIG. 5.

The hook tapes 12a and 12b and the target tape 40 are separated, and the diaper 1 is laid-out in the state shown in FIG. 1. Then, as shown in FIG. 7(a), the middle portion of the ventral region F in the width direction D2 is brought into contact with the belly of the male dog 2, and the diaper 1 is arranged so that the buttock covering region M is straddled by the two legs 2b of the male dog 2. At this time, the skin surface is brought into contact with the male dog 2.

Next, as shown in FIG. 7(b), the dorsal region R is lifted and folded so that the skin surface is inward in the buttock covering region M. Then, the tail 2c of the male dog 2 is passed through the tail opening 27, and the dorsal region R is brought into contact with the back of the male dog 2.

Then, as shown in FIG. 7 (c), both ends of the ventral region F in the width direction D2 are lifted and brought into contact with the dorsal region R so that the belly of the male dog 2 is wrapped by the ventral region F. Thus, the trunk 2a of the male dog 2 is passed through the waist opening WO formed by the ventral region F and the dorsal region R, and the legs 2b of the male dog 2 are passed through the pair of leg openings LO formed by the leg circumference region FB of the ventral region F and the buttock covering region M.

The hook tapes 12a and 12b are attached to both ends of the belly wrap region FA of the ventral region F in the width direction D2, and a target tape 40 is attached to the dorsal region R of the back sheet 21. Therefore, the male dog 2 can wear the diaper 1 by joining the hook tapes 12a and 12b and the target tape 40. At this time, the target tape 40 is attached to the entire area in the width direction D2 of the back sheet 21. Thus, the hook tapes 12a and 12b can be joined to any position in the width direction D2 of the dorsal region R, and the waist opening WO can be easily adjusted according to the waist of the pet.

In this way, the diaper 1 shown in FIG. 1 can also be worn by the pet as a tape type in which the diaper 1 is fixed by tape.

### (Effect When Used on a Male Pet)

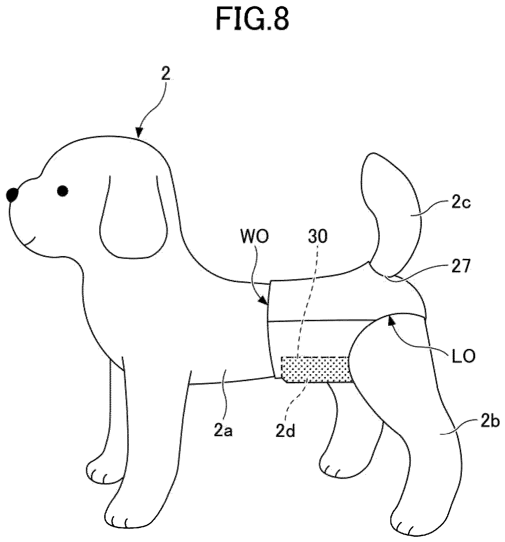

FIG. 8 is a diagram for explaining the effect when the diaper 1 shown in FIG. 1 is used on a male pet.

As described above, the absorber 30 is preferably arranged so that the distance d1 from the end portion 31 of the absorber 30 on the ventral region F side to the end portion 14a of the diaper 1 on the ventral region F side is in a range of 5 mm to 50 mm. Furthermore, it is more preferable that the absorber 30 is arranged so that this distance d1 is in a region of 10 mm to 40 mm. Thus, as shown in FIG. 8, when the male dog 2 with the urination opening 2d located on the ventral side wears the diaper 1, the absorber 30 covers the urination opening 2d of the male pet, so that the male pet can be accommodated.

At this time, the first region R1 overlapping the absorber 30 does not extend or contract in the width direction D2 in the belly wrap region FA of the exterior body 10. With this configuration, the absorber 30 located in the first region R1 does not adhere closely to the abdomen of the male dog 2 and is in a loose state. Thus, a urine capturing space is formed in which the absorber 30 surrounds the urination opening 2d of the male dog 2 at a distance from each other. Therefore, urine can be sufficiently captured by the absorber 30 and leakage of urine to the outside can be suppressed. Moreover, since the urination opening 2d and the absorber 30 are separated from each other, urine absorbed by the absorber 30 can be prevented from contacting and adhering to the urination opening 2d. Further, it is preferable that the rising end portions 67a and 67b of the three-dimensional gathers 60a and 60b are positioned within a range of 0 mm to 5 mm farther toward the side of the ventral end portion 14a of the diaper 1 and within a range of 0 mm to 5 mm farther toward the side of the dorsal end portion 14b of the diaper 1 than the end portion 31 of the absorber 30 on the side of the ventral region F. As a result, when the male dog 2 wears the diaper 1, a deformed protrusion protruding from the ventral end portion 31 of the absorber 30 to the non-skin-side side is formed, so that the urine capturing space is formed further forward and the absorber 30 is effectively utilized to capture urine from the ventral end.

The tension of each extendable member 13 is preferably 2.0 cN to 10.0 cN. With the tension of the extendable member 13 being 2.0 cN to 10.0 cN, when the male pet wears the diaper 1, the height of the protruding portion which protrudes to the non-skin surface side can be made sufficiently high, and the absorber 30 can be sufficiently separated from the abdomen of the male pet. Thus, a urine capturing space of sufficient size is formed in the diaper 1, and leakage of urine to the outside can be further suppressed.

Further, it is preferable that the rising end portions 68a and 68b are positioned within a range of 100 mm to 200 mm from the back end portion 14b of the diaper 1 to the ventral end portion 14a side. Thus, when a female pet wears the diaper 1, the three-dimensional gathers 60a and 60b rise to the skin surface side from the ventral side of the female pet on both sides of the urination opening, so that leakage of urine to the outside can be suppressed even when the female pet wears the diaper 1. That is, the diaper 1 can suppress leakage of urine to the outside regardless of whether the male pet or the female pet wears the diaper 1.

### (Diaper Usage for Small Pets)

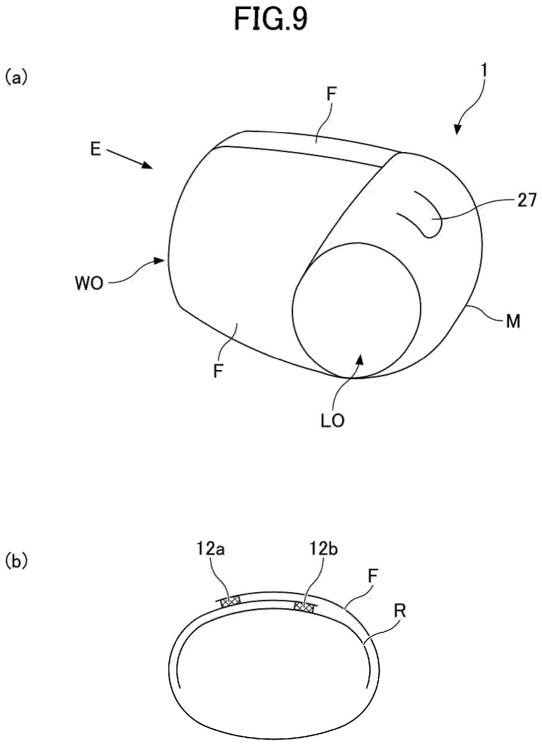

FIG. 9 is a view showing another usage mode of the diaper 1 shown in FIG. 1, wherein (a) is an external perspective view and (b) is a view from the direction of arrow E shown in FIG. 1.

The diaper 1 shown in FIG. 1 has the form shown in FIG. 5 and is used as a pants type, but it can also be used for small pets. In the diaper 1 shown in FIG. 1, the surface of the hook tapes 12a and 12b attached to the skin surface at respective ends of the ventral region F in the width direction D2 is made of a male material with a surface fastener. Therefore, it is possible to repeatedly engage with and separate from the exterior sheets 11a and 11b made of nonwoven fabrics. In other words, the hook tapes 12a and 12b are configured so that they can repeatedly engage with and separate from the belly wrap region FA where the hook tapes 12a and 12b are not attached.

Using this, for a small pet, as shown in FIG. 9, the dorsal region R is wrapped by the ventral region F, the hook tape 12b is joined to the target tape 40 of the dorsal region R, and the hook tape 12a is joined to the ventral region F where the hook tapes 12a and 12b are not attached. Thus, the diaper 1 can be used as a pants-type diaper 1 for a small pet as well.

When the tape type is used for a small pet, from the state shown in FIG. 7(a), the dorsal region R is wrapped by the ventral region F, the hook tape 12b is joined to the target tape 40 of the dorsal region R, and the hook tape 12a is joined to the ventral region F where the hook tapes 12a and 12b are not attached. Thus, the tape-type diaper 1 can be used for a small pet.

### (Other Embodiments)

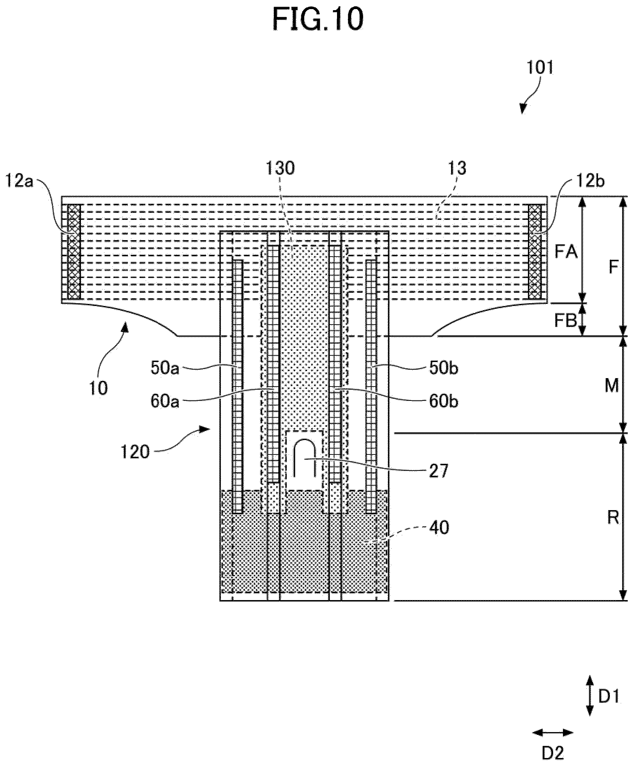

FIG. 10 is a view of another embodiment of the absorbent article for pets of the present disclosure viewed from the skin side, and is a plan view in a laid-out state.

As shown in FIG. 10, this embodiment is a diaper 101 in which the shape of an absorber 130 is different from that shown in FIG. 1.

The absorber 130 in this embodiment has a shape included not only in the ventral region F and the buttock covering region M, but also in the dorsal region R at the middle portion in the width direction D2 of the diaper 101. The absorber 130 is provided avoiding the tail opening 27.

The diaper 101 thus constructed has the same effect as that shown in FIG. 1, and can be used as a pants type or a tape type. However, as shown in FIG. 1, if the absorber 30 is arranged only in the ventral region F and the buttock covering region M, the cost can be reduced by not arranging the absorber 30 in the dorsal region R, where it is not expected to be effective.

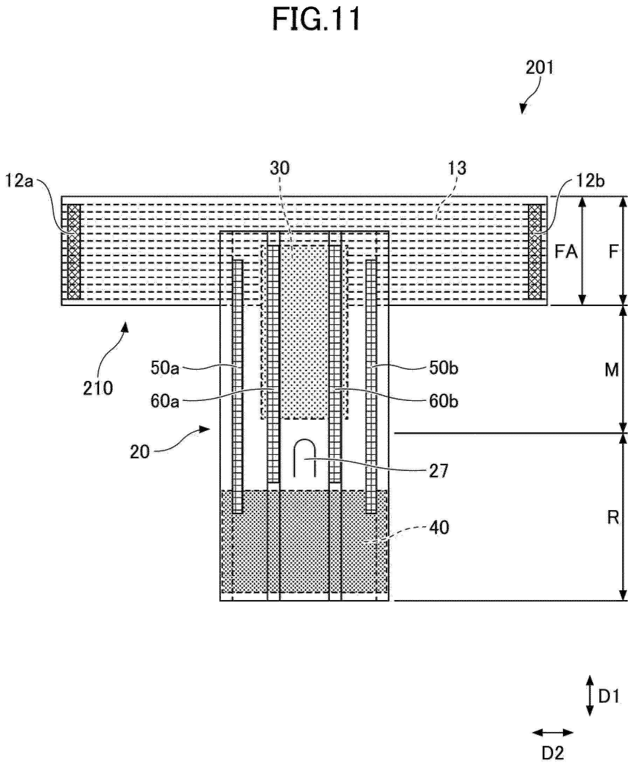

FIG. 11 is a view of another embodiment of the absorbent article for pets of the present disclosure viewed from the skin side, and is a plan view in a laid-out state.

As shown in FIG. 11, this embodiment is a diaper 201 in which the shape of an exterior body 210 is different from that shown in FIG. 1.

The exterior body 210 in this embodiment is composed only of a belt-like shape having the width direction D2 as the longitudinal direction. Thus, the ventral region F in this embodiment is composed only of the belly wrap region FA provided with the extendable members 13 and hook tapes 12a and 12b.

The diaper 201 thus constructed has the same effect as that shown in FIG. 1, and can be used as a pants type or a tape type. In the present embodiment, the ventral region F is composed of only the belly wrap region FA provided with the extendable members 13 and the hook tapes 12a and 12b. Thus, while constituting the pants type, the length of the ventral region F in the front-rear direction D1 is shortened to suppress the bulkiness of wearing the diaper 201, thereby improving the slimness and appearance.

This application claims priority to Japanese Patent Application No. 2023-026287, filed on February 22, 2023, and its entire contents are incorporated herein.

### [Description of Symbols]

1, 101, 201 Diaper
2 Male dog
2a Trunk
2b Leg
2c Tail
2d Urination opening
10, 210 Exterior body
11a, 11b Exterior sheet
11c, 62a, 62b Folded-back part
12a, 12b Hook tape
13, 51a, 51b, 65a, 65b Extendable member
14a, 14b, 31, 67a, 67b, 68a, 68b End portion
20 Interior body
21 Back sheet
22 Waterproof sheet
23 Top sheet
27 Tail opening
30, 130 Absorber
40 Target tape
50a, 50b Planar gather
60a, 60b Three-dimensional gather
61a, 61b Gather sheet
63, 64, 66 Adhesive
F Ventral region
FA Belly wrap region
FB Leg circumference region
LO Leg opening
M Buttock covering region
R Back region
WO Waist opening

## Claims

1. An absorbent article for pets, comprising:
a dorsal region configured to cover a dorsal side from a base of a tail;
a buttock covering region that is connected to the dorsal region in a first direction and configured to cover an anus and a vicinity on a lower side thereof below the base of the tail;
a ventral region that is connected to the buttock covering region on a side opposite the dorsal region in the first direction, and configured to cover an abdomen in front of the buttock covering region; and
an absorber disposed in a middle portion of at least the buttock covering region and of the ventral region in a second direction orthogonal to the first direction,
wherein the ventral region has a belly wrap region that is joined to the dorsal region at both ends in the second direction, and is provided with extendable members that each extend and contract in the second direction, over an entire area of the belly wrap region in the first direction, and joining members that are joined to the dorsal region and provided at respective ends of the belly wrap region in a longitudinal direction,
the dorsal region is provided with a member to be joined to the joining members,
the joining members and the member to be joined are configured to be capable of being joined to and separated from each other repeatedly,
the joining members are configured to be capable of being repeatedly joined to and separated from even a portion of the belly wrap region to which the joining members are not attached.

2. The absorbent article for pets according to claim 1, wherein the belly wrap region has a length of 80 mm or more in the first direction.

3. The absorbent article for pets according to claim 1, wherein the absorber is not disposed in the dorsal region.

4. The absorbent article for pets according to claim 3, wherein the absorber is disposed such that an end portion of the absorber on a ventral region side is within a range of 10 mm to 40 mm from an end portion of the absorbent article for pets on a ventral region side.

5. The absorbent article for pets according to claim 1, further comprising a pair of three-dimensional gathers that extend along respective sides of the absorber in the first direction and that rise toward a skin surface,
wherein a rising end portion of each of the three-dimensional gathers on a ventral region side is located within a range of 0 mm to 5 mm toward an end portion of the absorbent article for pets on a ventral region side or within a range of 0 mm to 5 mm toward an end portion of the absorbent article for pets on a dorsal region side, from an end portion of the absorber on a ventral region side.

6. The absorbent article for pets according to claim 5, wherein the rising end portion of the solid gather on a dorsal region side is located 100 mm to 200 mm closer to the end portion of the absorbent article for pets on the ventral region side than the end portion of the absorber on a dorsal region side is.

7. The absorbent article for pets according to claim 1,
wherein the belly wrap region includes a first region overlapping the absorber and a second region not overlapping the absorber, and
wherein the second region is extended and contracted in the second direction by the extendable members, and the first region is not extended and contracted in the second direction.

8. The absorbent article for pets according to claim 1, wherein the extendable members are capable of extending to be 1.5 to 3.5 times the length of the extendable members when contracted, in the second direction of the belly wrap region.

9. The absorbent article for pets according to claim 1, wherein the ventral region includes only the belly wrap region provided with the extendable members and the joining members.

10. The absorbent article for pets according to claim 1, wherein the joining members are provided over an entire area of the dorsal region in the second direction.

11. The absorbent article for pets according to claim 1, wherein the joining members are joined to the member to be joined, to form a pants type.

12. The absorbent article for pets according to claim 10, wherein the joining members are joined to a region, of the member to be joined, corresponding to the body shape of a pet wearing the absorbent article for pets, to form a pants type.
